Europäisches Patentamt

European Patent Office

Office européen des brevets

(11) Publication number: **0 425 666 B1**

# EUROPEAN PATENT SPECIFICATION

(45) Date of publication of patent specification: **13.04.94**

(51) Int. Cl.5: **C07C 57/05**, C07C 57/04, C07C 51/215, C07C 47/22, C07C 27/14, B01J 23/28

(21) Application number: **89905775.6**

(22) Date of filing: **22.05.89**

(86) International application number: **PCT/JP89/00509**

(87) International publication number: **WO 90/14325 (29.11.90 90/27)**

(54) **PROCESS FOR PRODUCING METHACRYLIC ACID AND METHACROLEIN.**

(43) Date of publication of application:
**08.05.91 Bulletin 91/19**

(45) Publication of the grant of the patent:
**13.04.94 Bulletin 94/15**

(84) Designated Contracting States:
**DE FR GB IT**

(56) References cited:
**JP-A- 5 283 306**
**JP-A- 5 562 041**
**JP-A-62 132 832**
**JP-A-63 145 249**
**JP-B-51 664 7**

(73) Proprietor: **Asahi Kasei Kogyo Kabushiki Kaisha**
**2-6, Dojimahama 1-chome**
**Kita-ku**
**Osaka-shi Osaka 530(JP)**

(72) Inventor: **YAMAMATSU, Setsuo**
**1434-15, Atsuhara**
**Fuji-shi, Sizuoka-ken 419-02(JP)**
Inventor: **YAMAGUCHI, Tatsuo**
**107-1, Maeda**
**Fuji-shi, Sizuoka-ken 416(JP)**

(74) Representative: **Strehl Schübel-Hopf Groening & Partner**
**Maximilianstrasse 54**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to a one-step process for the preparation of methacrylic acid and methacrolein, which comprises subjecting isobutane in the vapor phase to catalytic oxidation. More particularly, the present invention is concerned with a process for preparing methacrylic acid and methacrolein in one step, which comprises subjecting isobutane in the vapor phase to catalytic reaction in the presence of molecular oxygen at a high temperature, using a specific catalyst. The catalyst comprises a composition characterized by the system of P and/or As - Mo - V and/or Cu-alkali metal and/or alkaline earth metal and/or Tl, which composition contains a heteropoly acid. By the present process, the desired products are obtained in high yield and with high selectivity in one step, while maintaining the activity of the catalyst stably for a prolonged period of time.

Background Art

Heretofore, a saturated hydrocarbon, such as isobutane, has been regarded as being an inert gas. For example, in Japanese Patent Application Laid-open Specification No. 55-2619, there is a description to the effect that isobutane is used as a diluent for a reaction gas in the oxidation of an olefin or an aldehyde. Since isobutane is less reactive, as seen from the above, the conventional processes for the preparation of methacrolein or methacrylic acid from isobutane (see for example, Japanese Patent Application Laid-open Specification No. 58-189130) generally consists in the converting of isobutane to isobutylene by the use of a dehydrogenating catalyst or an oxidative dehydrogenating catalyst, and oxidizing the formed isobutylene to obtain the desired product.

In recent years, researches have been carried out with a one-step conversion process by the oxidation of isobutane to a valuable compound such as methacrolein and methacrylic acid. In British Patent No. 1340891, although the yield is extremely low, there is a description suggesting a one-step process for the production of methacrolein from isobutane by contacting a mixed gas of isobutane and oxygen in the vapor phase with an oxide comprising antimony, molybdenum, etc.

U.S. Patent No. 4,260,822 discloses a one-step process for the production of methacrylic acid from isobutane. In this process, a catalyst comprising oxides of antimony, molybdenum and phosphorus is used. For example, when the concentration of isobutane is 10 mol%, and the reaction temperature 340°C, 10 % of the isobutane participates in the reaction, 50 % of which is converted to methacrylic acid and 20% to methacrolein. The U.S. patent teaches that the use of a catalyst comprising oxides of antimony, molybdenum and phosphorus is requisite for obtaining methacrylic acid with high selectivity. When the reaction is performed at an increased isobutane concentration of 28 mol%, 9% of the isobutane is converted with a 46% selectivity to methacrylic acid and a 10% selectivity to methacrolein. The process of U.S. Patent No. 4,260,822 is unsatisfactory as a commercial process from the viewpoint of productivity per unit weight of the catalyst.

Thereafter, in Japanese Patent Application Laid-Open Specification No. 62-132832, a process for preparing methacrylic acid in high yield was proposed, which comprises reacting isobutane in a concentration as high as 30 to 60 mol%, using as the catalyst a heteropoly acid having phosphorus as a central element and containing molybdenum. In this process, an extremely characteristic reaction system is employed, in which isobutane and oxygen are alternately contacted with the catalyst. However, a special reaction apparatus is necessary for alternately contacting isobutane and oxygen with the catalyst, and the operation of such an apparatus is complicated. To cope with this problem, Japanese Patent Application Laid-Open Specification No. 63-145249 suggests the use of the above-mentioend catalyst in a highly reduced state. By the use of such a catalyst, the conversion to methacrylic acid can be performed with high selectivity even in a reaction mode in which a mixed gas of isobutane and oxygen is contacted with the catalyst. However, the catalyst is likely to suffer from gradual oxidation as the catalyst is used in the reaction over a prolonged period of time. Due to the oxidation, the activity of the catalyst is decreased and the selectivity for methacrylic acid is markedly lowered. Therefore, a catalyst of excellent durability which is capable of exhibiting a desirable reaction performance stably for a prolonged period of time, has been desired in the art.

In addition, the conventional processes in which a molybdenum heteropoly acid is used as a catalyst have a common drawback in that the reaction is carried out at a temperature of from 350° to 370 °C despite the fact that it is known that the molybdenum heteropoly acid decomposes at a temperature higher than 350 °C. This is because the activity of the catalyst is low and hence the reaction performance is poor at low temperatures. Actually, decomposition of a heteropoly acid occurs even at about 330 °C in the above reaction. Therefore, a catalyst having an excellent heat stability and capable of exhibiting excellent reaction performance even at a temperature of 350 °C or lower, and preferably 320 °C or lower, has been desired in the art.

More specifically, with respect to a process for preparing methacrylic acid by contacting a mixed gas of isobutane and oxygen in the vapor phase with a catalyst, an improved process which can be carried out at a temperature of 350 °C or lower, preferably 320 °C or lower, using a catalyst having an excellent heat stability and capable of exhibiting an excellent reaction performance even in an oxidized state with high selectivity and in high yield while maintaining a stable activity of the catalyst over a prolonged period of time, has been strongly desired.

Disclosure of The Invention

In the above-described situations, the present inventors have made extensive and intensive studies on heteropoly acid catalysts with a view toward developing a catalyst of excellent durability exhibiting an excellent reaction performance even at a temperature of about 320 °C or lower. As a result, it has been found that a catalyst comprising a composition characterized by the system of P and/or As - Mo - V and/or Cu-alkali metal and/or alkaline earth metal and/or Tl and containing a heteropoly acid having P and/or As as a central element or central elements and Mo as a coordinating element, which composition has been selected from among a vast plurality of elements and an almost infinite number of combinations, is a catalyst of excellent duration which can advantageously maintain the catalytic activity and selectivity for methacrylic acid for a prolonged period of time. Also, it has been found that methacrylic acid can be produced with high selectivity and in high yield even at temperatures as low as about 320 °C by the use of the catalyst. Furthermore, it has been found that even when the catalyst has been oxidized to decrease the degree of reduction to a level corresponding to an increase of two electrons or less per heteropoly acid, the catalyst exhibits an excellent reaction performance which is substantially equal to that exhibited when the catalyst is in a highly reduced state. The present invention has been completed on the basis of these findings.

According to the present invention, a process for the preparation of methacrylic acid and methacrolein is provided, which comprises contacting at a temperature of from 240° to 350 °C a mixed gas comprising isobutane and molecular oxygen in the vapor phase with a catalyst comprising a composition containing a heteropoly acid having P and/or As as a central element or central elements and Mo as a coordinating element. The composition is represented by the formula:

$$A_a \, Mo_{12} \, B_b \, C_c \, D_d \, O_e \qquad (1)$$

wherein A represents P and/or As; Mo represents molybdenum; B represents V and/or Cu; C represents at least one member selected from the group consisting of an alkali metal, an alkaline earth metal and Tl; D represents at least one member selected from the group consisting of Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr and Nd; O represents oxygen; and a, b, c, d and e are numbers respectively representing relative atomic proportions of A, B, C, D and O, wherein a, b, c and d are, respectively, in the ranges of from 0.5 to 3, from 0.01 to 3, from 0.01 to 3 and from 0 to 3, and e is the number of oxygens required to satisfy the valence and relative atomic proportion requirements of the elements present.

In the present invention, it is of critical importance to use the catalyst having the above-defined composition. When a catalyst lacks any of the essential components of the above-mentioned composition, or when a catalyst has relative atomic proportions which are outside the ranges defined above, the activity of the catalyst and the selectivity for methacrylic acid markedly drop as the catalyst is used in reaction over a prolonged period of time, as apparent from Comparative Examples 1 to 4 described later. By contrast, the catalyst to be used in the present invention has extremely excellent durability, and hence by the use of the catalyst it is possible to produce methacrylic acid in high yield and with high selectivity stably over a prolonged period of time. This catalyst not only has excellent durability but also exhibits a high reactivity. Due to the high reactivity, the reaction temperature can be lowered, and it is possible to carry out the reaction at a temperature at which thermal decomposition of the heteropoly acid is unlikely to occur. The low reaction temperature has a favorable effect on the further improvement of the durability of the catalyst. The above-mentioned excellent durability and high reactivity of the catalyst to be used in the present invention would be attributed to the employment as catalyst constituent elements of vanadium and/or copper in combination with at least one member selected from the group consisting of an alkali metal, an alkaline earth metal and Tl. Whilst the mechanism of the activity of such elements is now being investigated, it is believed that the alkali metal, alkaline earth metal and Tl are not only contributory to substantial improvement of the heat stability of the catalyst but are useful for activating the inert C-H bond of isobutane. In conventional processes, these basic components often strongly adsorb methacrylic acid as a reaction product and cause the product to suffer from excess oxidation. However, in the process of the present invention, since such basic components are used in combination with vanadium and/or copper, the formation, on the catalyst, of an oxygen seed which causes excess oxidation, can be effectively inhibited. As a result, the isobutane conversion is improved with the selectivity for methacrylic acid maintained at a high value.

According to the process of Japanese Patent Application Laid-Open Specification No. 63-145249, it is inevitably necessary to use a catalyst having a high degree of reduction. By contrast, according to the present invention, it is not essential to use a catalyst having a high degree of reduction. As substantiated in Example 1 described later, according to the present invention, an excellent reaction performance can be exerted stably over a prolonged period of time even when the catalyst has a low degree of reduction, e.g., a level corresponding to two-electron reduction or lower. It is believed that due to the above-mentioned lower tendency of formation, on the catalyst, of an oxygen seed, which is the cause of excess oxidation, and the excellent heat stability of the catalyst, the catalyst to be used in the present invention is active even when the state of the catalyst is near a fully oxidized state. This means that the catalyst to be used in the present invention can be used stably for a prolonged period of time despite a reaction atmosphere containing oxygen in which the catalyst is likely to suffer from oxidation. By this unexpected finding, it has been realized to provide a process in which a mixed gas of isobutane and oxygen in the vapor phase is contacted with a catalyst to produce methacrylic acid in high yield and with high selectivity stably for a prolonged period of time, even though the catalyst is exposed to oxygen.

Moreover, it is noted that successive oxidation of reaction products, such as methacrylic acid and methacrolein, which leads to the formation of carbon dioxide etc. is effectively suppressed by the lowering of the reaction temperature, so that the selectivity for methacrylic acid and methacrolein can be improved. In particular, when the reaction is performed in a fluid bed reactor, the reaction products are likely to suffer from excess oxidation, leading to a lowering in the selectivity for a methacrylic acid as compared to the case in which a fixed bed reaction system is used. However, in the process of the present invention, a high selectivity for methacrylic acid is obtained even in the case in which a fluid bed reactor is employed since excess oxidation is effectively suppressed in the process of the present invention.

Hereinbelow, the present invention will be explained in more detail. It is of critical importance that the catalyst to be employed in the present invention contain a heteropoly acid having P and/or As as a central element or central elements and Mo as a coordinating element and also contain V and/or Cu and at least one member selected from the group consisting of an alkali metal, an alkaline earth metal and Tl. Examples of alkali metals include Li, Na, K, Rb and Cs, which can be used individually or in combination. Examples of alkaline earth metals include Mg, Ca, Sr and Ba, which can be used individually or in combination. With respect to the constituent elements of the catalyst, the relative atomic proportion of P and/or As is in the range of from 0.5 to 3 and that of V and/or Cu is in the range of from 0.01 to 3, and preferably from 0.05 to 2.0, relative to 12 atoms of Mo. At least one member selected from the group consisting of the alkali metal, the alkaline earth metal and Tl is used in a relative atomic proportion of from 0.01 to 3, and preferably from 0.1 to 2. Further, when at least one member selected from the group consisting of Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr and Nd is used, the relative atomic proportion thereof is preferably in the range of from 0.01 to 3, and more preferably from 0.05 to 1.

Examples of heteropoly acids having P and/or As as a central element or central elements and Mo as a coordinating element include a molybdophosphoric acid, an molybdoarsenic acid, a molybdoarsenophosphoric acid, and the like, which are known to assume various structures (see Sasaki and Matsumoto: Kagaku no Ryoiki, vol. 29, no. 12, page 853). For example, heteropoly acids having structures in which the ratios of central element to coordinating element are, respectively, 1/12, 1/11, 1/10, 1/9, 2/17 and 2/18 are known. Among the heteropoly acids, one having a structure in which the above-mentioned ratio is 1/12, known as the Keggin structure, is especially preferred.

It is preferred that vanadium be present in a form such that it substitutes for a portion of the coordinating element of a molybdophosphoric acid, an molybdoarsenic acid or a molybdoarsenophosphoric acid. However, a portion of the vanadium may be present in the form of a non-heteropoly acid, such as an oxide or an oxyacid. The chemical state of the copper present in the catalyst is extremely complicated, and has not yet been elucidated in detail. Copper may be present either in the form of a salt with a heteropoly acid or in the form of a non-heteropoly acid, such as an oxide and an oxyacid. Alternatively, copper may be present in a form such that it substitutes for a portion of the elements constituting the heteropoly acid. It is preferred that copper be present in the form of an oxide or a salt of a heteropoly acid. With respect to an alkali metal, an alkaline earth metal and Tl, these are mainly present in the form of a salt of a heteropoly

4

acid. Further, as in the case of Cu described above, Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr and Nd may be present either in the form of a salt of a heteropoly acid or in the form of a non-heteropoly acid, such as an oxide and an oxyacid. Alternatively, each of these elements may be present in a form such that it substitutes for a portion of the elements constituting the heteropoly acid.

The catalyst to be employed in the present invention can readily be prepared according to the conventional methods (preparative operations). Generally, the catalyst is prepared by first adding compounds containing necessary elements, which are in the form of an oxide, a hydroxide, a carbonate, a nitrate, a chloride, an oxyacid, a phosphate, an oxalate, an acetate, an organic complex compound or a metal, to a solution or a slurry of a heteropoly acid, such as a molybdophosphoric acid, a molybdovanadophosphoric acid, a molybdoarsenic acid or a molybdovanadoarsenic acid, or a salt thereof, or to a place under conditions in which such a heteropoly acid is formed, subsequently performing evaporation to dryness, and finally calcining the resultant solid in an air environment at 250° to 500 °C for 2 to 24 hours. However, the method of catalyst preparation and the starting materials for use in catalyst preparation are not limited to the above. Other methods and starting materials may be used. For example, various nitrogen-containing compound salts of a heteropoly acid may be used as a starting material. Representative examples of effective salts include ammonium salts, and salts with organic amines, such as pyridine, quinoline and piperadine. These nitrogen-containing compounds may be used in the step of catalyst preparation. With respect to the ammonium salts, aqueous ammonia or water soluble ammonium salts, such as ammonium chloride and ammonium nitrate, can be added as an ammonium ion source. These ammonium salts or organic amine salts are calcined at from 300° to 600 °C to remove a portion or all of the nitrogen-containing compound, prior to use as a catalyst. Calcination is preferably performed in an inert gas. Calcination in an inert gas may be followed by calcination in an oxygen-containing gas.

These catalysts may be carried on a carrier or may be diluted and mixed with a carrier. Representative examples of carriers include silica, α-alumina, titania, zirconia, diatom earth, silica alumina, water soluble silica sol and silicon carbide. Inert carriers having a vast plurality of macropores and thus a high porosity are preferred. The catalyst is generally carried on or mixed with such a carrier, in the presence or absence of water, in an amount of up to the weight equal to the weight of the carrier.

Prior to use, the catalyst may preferably be molded into a suitable shape and a suitable size, e.g., pellet or granular shape, depending on application conditions, so as to impart a desired mechanical strength to the catalyst. In molding the catalyst, a tablet machine, an extrusion molding machine, Marumerizer (trade name of a product of Fuji Paudal, Japan), a rolling type granulator and the like may be used.

The degree of reduction at which the catalyst functions under the reaction conditions for the process fo the present invention has not completely been elucidated. However, since even the catalyst in a state of high degree of reduction often turns into a yellow-greenish color with the lapse of reaction time, not exhibiting a dark blue color known as heteropoly blue, the catalyst under the reaction conditions is considered to be in a state of a degree of reduction as low as a level corresponding to 1 or 2 electron increase per heteropoly acid or lower. The degree of reduction, however, greatly depends on the composition of the catalyst, the composition of the reaction gas and the reaction temperature. Therefore, the degree of reduction of the catalyst is not limited to the above range.

As a gaseous feedstock to be supplied to the reaction system, a mixed gas comprising isobutane and oxygen is used. The isobutane content of the mixed gas is preferably in the range of from 10 to 80 % by mole. If the isobutane content is lower than 10 % by mole, the amount of methacrylic acid produced per reactor is so small that productivity suited for commercial production cannot be obtained. The isobutane content is more preferably in the range of from 20 to 60 by mole. Isobutane to be used in the catalytic reaction is not necessarily of high purity, and hence a mixture in which a paraffinic hydrocarbon not adversely affecting the reaction is contained in a molar amount of about two times that of isobutane may be employed. It is preferred to use isobutane isolated by distillation from LPG butane, FCC butane or a reaction product of isomerization of n-butane. If an olefin is contained in a molar amount of about 0.1 time or more that of isobutane, the formation of by-products is increased. Hence, contamination with olefins other than isobutylene is preferably to be avoided.

In the present invention, the molar ratio of oxygen to isobutane is preferably in the range of from 0.05:1 to 1:1, more preferably in the range of from 0.1:1 to 0.6:1. If the molar proportion of oxygen is higher than the above, complete oxidation occurs excessively, resulting in an increase in the formation of carbon dioxide. On the other hand, if the molar proportion of oxygen is smaller than the above, oxygen supply is not sufficient for oxidation of isobutane and the conversion of isobutane is lowered. Also, if the molar proportion of oxygen is smaller than the above, the reduction of the catalyst disadvantageously proceeds in excess with the advance of the reaction.

It is preferred that the oxygen content and isobutane content be chosen so that the composition of the resultant mixed gas may be outside an explosive range. As the molecular oxygen to be used for the catalytic reaction, pure oxygen gas may be employed. However, since it is not essential to use a molecular oxygen of high purity, the use of air is generally preferred from the economical point of view. Further, it is possible to prevent the composition of the mixed gas from falling inside the explosive range, by using, as a diluent for the mixed gas, nitrogen and other inert gases having no adverse effect on the reaction, such as helium, argon and carbon dioxide. In this case, the inert gases are generally used at a molar ratio to isobutane of from 1:10 to 10:1.

In order to prevent methacrylic acid as a reaction product from being further oxidized on the catalyst and thereby forming carbon dioxide and the like, steam is preferably added to molecular oxygen and isobutane, at a molar ratio to isobutane of from 1:5 to 5:1, thereby enhancing the selectivity for methacrylic acid. The molar ratio is more preferably in the range of from 1:3 to 3:1.

The reaction temperature is chosen in the range of from 240° to 350 °C, preferably 270° to 320 °C. If the reaction temperature is higher than the above, the decomposition of the catalyst and the complete oxidation of the reaction product are likely to occur. By virtue of the catalyst used in the present invention, a high selectivity for methacrylic acid and a high catalytic activity are attained even when the reaction temperature is 320 °C or lower.

The reaction pressure may be selected in a wide range from a reduced pressure to a super-atmospheric pressure. However, the pressure which is advantageous from the viewpoint of commercial production is in the range from atmospheric pressure to about 1MPa.

The contact time (i.e., the value obtained by dividing the bulk volume of the catalyst by the volume gas flow rate of the mixed gas at the reaction temperature and the reaction pressure) of the mixed gas as a feedstock with the catalyst varies depending on the isobutane concentration and the reaction temperature. The contact time is generally from 0.1 to 10 seconds, preferably from 0.5 to 5 seconds.

In the practice of the present invention, the reactor to be used can suitably be selected from various types including a fixed bed reactor, a fluidized bed reactor and a moving bed reactor.

Methacrylic acid and methacrolein formed can be isolated from by-produced oxides by a series of customary processes, such as cooling, extraction and distillation, and purified by any of customary methods. When unreacted isobutane is recovered and recycled into the reactor, the recycled isobutane may contain reaction products, such as carbon monoxide and carbon dioxide, as long as the content of these products does not adversely affect the subsequent reaction. By feeding back methacrolein together with the feedstock mixed gas containing isobutane into the reactor, the methacrolein can be effectively converted into methacrylic acid.

Best Mode for Carrying Out the Invention

The present invention will be described in more detail with reference to the Examples, which should not be construed to be limiting the scope of the present invention.

[Examples]

Example 1

144.0 g of molybdenum trioxide, 8.27 g of vanadium pentoxide and 12.5 g (85 % by weight) of phosphoric acid were charged into a three-neck Pyrex flask together with 1000 ml of water, and heated under reflux for 24 hours. Then, the insoluble matter in the aqueous solution was filtered off, and the solution was concentrated, thereby obtaining red-brown crystals. By analysis using X-ray diffraction, atomic absorption spectroscopy and $^{31}$P-NMR, the crystals were found to be molybdovanadophosphoric acid (PMo$_{11}$V) having P:Mo:V relative atomic proportions of 1:11:1. The crystals were in the form of approximately triaconta hydrates. 23.2 g of the crystals and 0.1 g of cuprous chloride were dissolved in 200 ml of water heated to 80 °C. 2.0 g of cesium nitrate dissolved in 50 ml of water was added thereto, and further 23.8 g of pyridine dissolved in 100 ml of water was added thereto, thereby obtaining a slurry. The slurry was concentrated and then dried at 120 °C for 12 hours. The resultant solid product was pulverized and particles of 10 to 20 mesh were selected, followed by calcining at 450 °C for 3 hours under a stream of nitrogen. As a result, a catalyst having a composition of $P_{1.1}Mo_{12}V_{1.1}Cu_{0.11}Cs_{1.1}$ (showing relative atomic proportions excluding that of oxygen being indicated; this being applicable to the formulae shown hereinafter) was obtained.

5 g of the catalyst was introduced into a Pyrex U-tube having an inner diameter of 6 mm and set in a thermostatic bath. The temperature of the thermostatic bath was set at 320 °C, and a mixed gas consisting of 30 % by mole of isobutane, 15 % by mole of oxygen, 20 % by mole of steam and 35 % by mole of nitrogen was fed at a contact time of 3.6 seconds. When the reaction gas was analyzed by gas chromatography 100 hours after the beginning of the reaction, it was found that 10.3 % of the isobutane was converted and that the selectivity for methacrylic acid was 55.7 % and the selectivity for methacrolein was 16.3 %. No isobutylene was detected. At this stage, the reduction degree of the catalyst corresponded to an increase of 2.5 electrons per heteropoly acid. The reaction was performed for 1000 hours under substantially the same conditions. As a result, 10.1 % of the isobutane was converted, and the selectivity for methacrylic acid was 56.3 % and the selectivity for methacrolein was 15.9 %. At this stage, the reduction degree of the catalyst corresponded to an increase of 0.3 electron per heteropoly acid.

Example 2

200 ml of water was heated to 80 °C. 23.5 g of 12-molybdophosphoric acid ($H_3PMo_{12}O_{40}.30H_2O$; produced by Nippon Inorganic Colour & Chemical Co., Ltd., Japan) was dissolved therein, and, while stirring, a solution prepared by dissolving 1.0 g of potassium nitrate and 0.48 g of copper nitrate in 20 ml of water and further 1.17 g of ammonium metavanadate were added. Then, to the resultant solution was added an aqueous solution prepared by dissolving 6.4 g of ammonium nitrate in 100 ml of water. The thus obtained slurry was concentrated by heating while stirring and subjected to evaporation to dryness on a evaporating dish, followed by further drying at 120 °C for 12 hours. Thus, a solid product was obtained. The solid product was pulverized, and particles of 10 to 20 mesh were collected and calcined at 450 °C for 3 hours under a stream of nitrogen, thereby obtaining a catalyst. The catalyst had a composition of formula: $P_1Mo_{12}V_1Cuo_{0.2}K_1$.

Of the catalyst, 5 g was taken and charged into a Pyrex U-tube having an inner diameter of 6 mm. The tube was placed in a thermostatic bath, and the temperature of the thermostatic bath was adjusted to 300 °C. A mixed gas of 25 % by mole of isobutane, 55 % by mole of air and 20 % by mole of steam was fed at a contact time of 3.6 seconds. When the gaseous reaction mixture was analyzed by gas chromatography 100 hours after the beginning of the reaction, it was found that 7.8 % of the isobutane was converted, and that the selectivity for methacrylic acid was 53.4 % and the selectivity for methacrolein was 20.8 %. No isobutylene was detected. At this stage, the reduction degree of the catalyst corresponded to an increase of 1.2 electrons per heteropoly acid. The reaction was performed for 1000 hours under substantially the same conditions. As a result, 7.5 % of the isobutane was converted, and the selectivity for methacrylic acid was 54.4 % and the selectivity for methacrolein was 21.1 %. At this stage, the reduction degree of the catalyst corresponded to an increase of 0.3 electron per heteropoly acid.

Example 3

23.5 g of 12-molybdophosphoric acid ($H_3PMo_{12}O_{40}.30H_2O$: produced by Nippon Inorganic Colour & Chemical Co., Ltd., Japan) was dissolved in 200 ml of water. To the resultant solution were added 0.37 g of sodium metavanadate and 1.15 g of $3As_2O_5 \cdot 5H_2O$ while stirring, and then the mixture was stirred at 60 °C for 12 hours. To the resultant solution were added a solution prepared by dissolving 0.5 g of rubidium nitrate and 1.33 g of thallium nitrate in 20 ml of water and an aqueous solution prepared by dissolving 12.8 g of ammonium nitrate in 100 ml of water in this order. The mixture was stirred to obtain a slurry. The thus obtained slurry was concentrated while heating and stirring, then evaporated to dryness using an evaporating dish, and dried at 120 °c for 12 hours to obtain a solid. The solid was pelletized under a pressure of about 100 kg/cm², and then pulverized. From the resultant powder, particles of from 10 to 20 mesh were collected. The particles were calcined under a nitrogen stream at 450 °C for 3 hours to obtain a catalyst. The catalyst had a composition represented by the formula: $P_1Mo_{12}V_{0.3}As_{0.64}Rb_{0.5}Tl_{0.5}$.

5 g of the catalyst was introduced into a pyrex U-tube having an inner diameter of 6 mm, and the U-tube was set in a thermostatic bath. While maintaining the temperature of the bath at 340 °C, a mixed gas consisting of 30 % by mole of isobutane, 50 % by mole of air and 20 % by mole of steam was fed into to the U-tube at a flow rate such that the contact time of the gas with the catalyst was 3.6 seconds. 100 Hours later, the gaseous reaction mixture was subjected to analysis by means of gas chromatography. It was found that 10.8 % of isobutane was converted, and that the selectivity for methacrylic acid and the selectivity for methacrolein were 45.8 % and 18.2 %, respectively. No isobutylene was detected. At this stage, the reduction degree of the catalyst corresponded to an increase of 2.1 electrons per heteropoly acid. A reaction was separately performed under substantially the same conditions as above except that the reaction time was changed to 500 hours. It was found that 9.9 % of isobutane was converted, and that the selectivity for methacrylic acid and the selectivity for methacrolein were 47.2 % and 19.3 %, respectively. At this stage, the reduction degree of the catalyst corresponded to an increase of 0.95 electron per heteropoly acid.

Example 4

23.5 g of 12-molybdophosphoric acid ($H_3PMo_{12}O_{40}$.30$H_2O$: produced by Nippon Inorganic Colour & Chemical Co., Ltd., Japan) was dissolved in water. To the resultant solution was added 0.85 g of arsenic acid ($H_3AsO_4$), followed by stirring at 60 °C for 12 hours. To the resultant solution were added 0.24 g of copper nitrate and a solution prepared by dissolving 2.66 g of thallium nitrate and 1.31 g of barium nitrate in 40 ml of water in this order. Then, to the resultant solution was added a solution prepared by dissolving 13.7 g of pyridine in 100 ml of water. The resultant mixture was stirred to obtain a slurry. The thus obtained slurry was concentrated while heating and stirring, then evaporated to dryness using an evaporating dish, and dried at 120 °C for 12 hours to obtain a solid. The solid was pelletized under a pressure of about 100 kg/cm$^2$, and then pulverized. From the resultant powder, particles of from 10 to 20 mesh were collected. The particles were calcined at 450 °C for 3 hours to obtain a catalyst. The catalyst had a composition represented by the formula: $P_1Mo_{12}As_{0.6}Cu_{0.1}Tl_1Ba_{0.5}$.

5 g of the catalyst was introduced into a pyrex U-tube having an inner diameter of 6 mm, and the U-tube was set in a thermostatic bath. While maintaining the temperature of the bath at 340 °C, a mixed gas consisting of 60 % by mole of isobutane, 20 % by mole of oxygen and 20 % by mole of steam was fed into the U-tube at a flow rate such that the contact time of the gas with the catalyst was 3.6 seconds. 100 Hours later, the gaseous reaction mixture was subjected to analysis by means of gas chromatography. It was found that 9.5 % of isobutane was converted, and that the selectivity for methacrylic acid and the selectivity for methacrolein were 44.2 % and 17.4 %, respectively. No isobutylene was detected. At this stage, the reduction degree of the catalyst corresponded to an increase of 1.8 electrons per heteropoly acid. A reaction was separately performed for 500 hours under substantially the same conditions as above. It was found that 9.4 % of isobutane was converted, and that the selectivity for methacrylic acid and the selectivity for methacrolein were 43.3 % and 17.1 %, respectively. At this stage, the reduction degree of the catalyst corresponded to an increase of 0.8 electron per heteropoly acid.

Comparative Example 1

In substantially the same manner for catalyst preparation as in Example 1, a catalyst having a composition represented by the formula: $P_1Mo_{12}Cs_1$ was obtained. 12-Molybdophosphoric acid ($H_3PMo_{12}O_{40}$. 3O$H_2O$: produced by Nippon Inorganic Colour & Chemical Co., Ltd., Japan) was used instead of $PMo_{11}V$. Cuprous chloride was not used. The catalyst was calcined in an air at 350 °C for 2 hours. Then, catalytic reaction was performed under substantially the same reaction conditions as in Example 1. 20 Hours later, the resultant gaseous reaction mixture was subjected to analysis by means of gas chromatography. It was found that 5.8 % of isobutane was converted, and that the selectivity for methacrylic acid and the selectivity for methacrolein were 30.1 % and 24.5 %, respectively. At this stage, the reduction degree of the catalyst corresponded to an increase of 0.3 electron per heteropoly acid.

Comparative Example 2

In substantially the same manner for catalyst preparation as in Example 1, a catalyst having a composition represented by the formula: $P_{1.1}Mo_{12}V_{1.1}$ was obtained. Differing from Example 1, cuprous chloride and cesium nitrate were not used. Catalytic reaction was performed under substantially the same reaction conditions as in Example 1. 20 Hours later, the resultant gaseous reaction mixture was subjected to analysis by means of gas chromatography. It was found that 5.3 % of isobutane was converted, and that the selectivity for methacrylic acid and the selectivity for methacrolein were 41.1 % and 19.2 %, respectively. At this stage, the reduction degree of the catalyst corresponded to an increase of 3.2 electrons per heteropoly acid. A reaction was separately performed for 100 hours under substantially the same conditions as above. It was found that the conversion of isobutane was as low as 3.3 %, and that the selectivity for methacrylic acid and the selectivity for methacrolein were 33.2 % and 27.8 %, respectively. At this stage, the reduction degree of the catalyst corresponded to an increase of 1.8 electrons per heteropoly acid.

8

Comparative Example 3

In substantially the same manner for catalyst preparation as in Example 1, a catalyst having a composition represented by the formula: $P_1Mo_{12}Cu_{0.5}$ was obtained. 12-Molybdophosphoric acid ($H_3PMo_{12}O_{40} \cdot 3OH_2O$: produced by Nippon Inorganic Colour & Chemical Co., Ltd., Japan) was used instead of $PMo_{11}V$. Differing from Example 1, cesium nitrate was not used. Catalytic reaction was performed under substantially the same reaction conditions as in Example 1. 20 Hours later, the resultant gaseous reaction mixture was subjected to analysis by means of gas chromatography. It was found that 5.4 % of isobutane was converted, and that the selectivity for methacrylic acid and the selectivity for methacrolein were 42.1 % and 22.5 %, respectively. At this stage, the reduction degree of the catalyst corresponded to an increase of 2.6 electrons per heteropoly acid. A reaction was separately performed for 100 hours under substantially the same conditions as above. It was found that the conversion of isobutane was as low as 3.1 %, and that the selectivity for methacrylic acid and the selectivity for methacrolein were 31.8 % and 25.9 %, respectively. The reduction degree of the catalyst at this stage corresponded to an increase of 0.9 electron per heteropoly acid.

Comparative Example 4

In substantially the same manner for catalyst preparation as in Example 1, a catalyst having a composition represented by the formula: $P_1Mo_{12}V_{0.5}As_{0.1}Cu_{0.2}$ was obtained. 12-Molybdophosphoric acid ($H_3PMo_{12}O_{40} \cdot 30H_2O$: produced by Nippon Inorganic Colour & Chemical Co., Ltd., Japan) and arsenic acid ($H_3AsO_4$) were used in addition to the raw materials used in Example 1. Catalytic reaction was performed under substantially the same conditions as in Example 1 except that the reaction temperature was changed to 340 °C. 100 Hours later, the resultant gaseous reaction mixture was subjected to analysis by means of gas chromatography. It was found that 10.5 % of isobutane was converted and that the selectivity for methacrylic acid and the selectivity for methacrolein were 46.2 % and 19.5 %, respectively. At this stage, the reduction degree of the catalyst corresponded to an increase of 1.8 electrons per heteropoly acid. A reaction was separately performed for 100 hours under substantially the same conditions as above. It was found that the conversion of isobutane was as low as 3.2 %, and that the selectivity for methacrylic acid and the selectivity for methacrolein were 28.8 % and 27.3 %, respectively. The reduction degree of the catalyst at this stage corresponded to an increase of 0.3 electron per heteropoly acid.

Examples 5 to 17

In substantially the same manner for catalyst preparation as in Example 1, catalysts having the compositions shown in Table 1 were obtained. Reaction was performed in substantially the same manner as in Example 1 except that the reaction temperature was changed to 340 °C. The results after the reaction for 100 hours are shown.

Table 1

| Example | Composition of catalyst | Conversion of isobutane % | Selectivity for methacrylic acid % | Selectivity for methacrolein % |
|---|---|---|---|---|
| 5 | $P_{1.3}Mo_{12}V_{0.2}K_1$ | 11.2 | 45.8 | 15.4 |
| 6 | $P_{1.5}Mo_{12}V_{0.5}Cs_{0.2}$ | 9.5 | 47.4 | 18.2 |
| 7 | $P_1Mo_{12}V_{1.5}Mg_{0.3}$ | 9.2 | 47.2 | 19.3 |
| 8 | $P_2Mo_{12}V_2As_{0.1}Rb_{1.5}Sr_{0.1}$ | 10.9 | 46.2 | 17.3 |
| 9 | $P_{1.3}Mo_{12}V_1Cs_1Na_{0.1}$ | 11.6 | 44.3 | 14.8 |
| 10 | $P_{1.2}Mo_{12}V_{1.5}Cs_2$ | 9.0 | 46.8 | 16.3 |
| 11 | $P_{1.3}Mo_{12}Cu_{0.1}Rb_1$ | 12.4 | 44.8 | 17.6 |
| 12 | $P_{1.3}Mo_{12}Cu_{0.2}Tl_{0.5}Ca_{0.5}$ | 11.6 | 42.3 | 15.3 |
| 13 | $P_1Mo_{12}Cu_{0.5}Cs_{0.1}Rb_{0.5}$ | 10.0 | 44.3 | 18.9 |
| 14 | $P_{1.3}Mo_{12}Cu_{0.1}Li_{0.1}Rb_{0.3}$ | 13.7 | 41.2 | 12.5 |
| 15 | $P_2Mo_{12}Cu_1As_{0.6}Tl_{1.5}$ | 10.6 | 44.5 | 19.8 |
| 16 | $P_{1.3}Mo_{12}Cu_{0.1}As_{0.2}Cs_1$ | 10.8 | 46.2 | 17.2 |
| 17 | $P_{1.3}Mo_{12}Cu_{0.5}Mg_{0.5}Ba_{0.5}$ | 8.4 | 42.3 | 15.5 |

9

Comparative Examples 5 to 17

Substantially the same procedure for catalyst preparation process as in Example 1 was repeated to thereby obtain catalysts having the compositions shown in Table 2 which are outside the scope of the present invention. Catalytic reaction was performed under substantially the same conditions as in Example 1 except that, in certain Comparative Examples, the reaction temperature was changed as indicated in Table 2. The results after the reaction for 20 hours are shown in Table 2.

Table 2

| Compara-tive Example | Composition of catalyst | Reaction tempera-ture °C | Conver-sion of iso-butane % | Selec-tivity for meth-acrylic acid % | Selec-tivity for meth-acrolein % |
|---|---|---|---|---|---|
| 5 | $P_{1.3}Mo_{12}$ | 340 | 10.5 | 18.3 | 21.1 |
| 6 | $P_{1.3}Mo_{12}K_2$ | 340 | 7.3 | 34.3 | 15.3 |
| 7 | $P_{1.3}Mo_{12}Cs_{1.5}$ | 340 | 8.0 | 30.2 | 17.2 |
| 8 | $P_{1.3}Mo_{12}Li_1$ | 340 | 6.9 | 27.2 | 25.3 |
| 9 | $P_{1.3}Mo_{12}As_{0.2}Mg_1$ | 340 | 8.3 | 32.5 | 18.9 |
| 10 | $P_{1.3}Mo_{12}Pb_{0.2}K_1$ | 340 | 7.9 | 31.1 | 16.5 |
| 11 | $P_{1.3}Mo_{12}Cr_{0.5}Rb_1$ | 340 | 8.2 | 33.4 | 15.3 |
| 12 | $P_{1.3}Mo_{12}Zn_{0.2}Tl_1$ | 340 | 8.1 | 34.2 | 16.3 |
| 13 | $P_{1.3}Mo_{12}Sn_{0.4}Ca_{0.5}$ | 340 | 7.8 | 31.9 | 17.6 |
| 14 | $P_{1.3}Mo_{12}Te_{0.2}Sr_{0.5}$ | 340 | 8.4 | 34.5 | 18.9 |
| 15 | $P_{1.3}Mo_{12}V_{0.001}Cs_1$ | 320 | 5.9 | 38.2 | 19.3 |
| 16 | $P_{1.3}Mo_{12}Cu_{0.001}K_1$ | 320 | 6.2 | 36.3 | 20.1 |
| 17 | $P_1Mo_{12}V_{1.0}Cu_{1.0}$ | 320 | 9.3 | 40.5 | 18.3 |

Examples 18 to 31

Catalysts having the compositions shown in Table 3 were prepared. Reaction was performed in substantially the same manner as in Example 1. The results after the reaction for 100 hours are shown in Table 3.

10

Table 3

| Example | Composition of catalyst | Conversion of isobutane % | Selectivity for methacrylic acid % | Selectivity for methacrolein % |
|---|---|---|---|---|
| 18 | $P_{1.2}Mo_{12}V_1Ag_{0.25}K_1$ | 8.6 | 44.6 | 16.2 |
| 19 | $PMo_{12}V_{1.5}Ag_{0.1}As_{0.25}K_1$ | 10.2 | 46.5 | 18.2 |
| 20 | $P_{1.3}Mo_{12}V_1Sb_{0.1}Cs_1$ | 7.8 | 50.2 | 22.2 |
| 21 | $PMo_{12}V_{0.8}Zr_{0.3}Cs_1$ | 7.2 | 48.9 | 16.6 |
| 22 | $PMo_{12}V_1Te_{0.25}K_1$ | 7.6 | 50.2 | 18.2 |
| 23 | $PMo_{12}V_1Ta_{0.5}B_{0.1}Rb_1$ | 8.5 | 50.6 | 22.3 |
| 24 | $P_2Mo_{12}V_1Ge_{0.5}K_1$ | 8.2 | 45.6 | 19.8 |
| 25 | $PMo_{12}Cu_{0.1}As_{0.6}Rb_1$ | 7.0 | 45.6 | 17.2 |
| 26 | $PMo_{12}Cu_{0.2}Cr_{0.1}Cs_1$ | 7.1 | 44.3 | 20.2 |
| 27 | $PMo_{12}Cu_{0.5}Ti_{0.1}K_{0.5}$ | 6.9 | 43.8 | 21.2 |
| 28 | $PMo_{12}Cu_{0.2}Fe_{0.1}Sr_{0.2}K_1\,Cs_{0.5}$ | 6.8 | 46.2 | 19.8 |
| 29 | $PMo_{12}Cu_{0.1}Ni_{0.3}Ca_1Tl_{0.5}$ | 6.9 | 43.2 | 18.3 |
| 30 | $PMo_{12}Cu_{0.1}Co_{0.1}Y_{0.1}Cs_1$ | 7.2 | 45.6 | 19.6 |
| 31 | $P_{1.2}Mo_{12}Cu_{0.1}Sn_{0.1}Rb_{0.25}\,Cs_{0.25}Tl_{0.5}$ | 7.8 | 46.3 | 18.6 |

Examples 32 to 46

Catalysts having the compositions shown in Table 4 were prepared. Catalytic reaction was performed under substantially the same conditions as in Example 1. The results after the reaction for 100 hours are shown in Table 4.

Table 4

| Example | Composition of catalyst | Conversion of isobutane % | Selectivity for methacrylic acid % | Selectivity for methacrolein % |
|---|---|---|---|---|
| 32 | $PMo_{12}V_1Cu_{0.2}Tl_{0.5}Rb_{0.5}$ | 11.8 | 56.2 | 15.4 |
| 33 | $PMo_{12}V_{0.5}Cu_{0.2}Zn_{0.1}K_1$ | 10.9 | 52.4 | 16.8 |
| 34 | $PMo_{12}V_{1.5}Cu_{0.2}Rh_{0.1}Cs_1$ | 9.8 | 53.2 | 14.9 |
| 35 | $PMo_{12}V_1Cu_{0.5}Nb_1Mn_{0.5}Tl_1$ | 10.1 | 53.8 | 17.2 |
| 36 | $PMo_{12}V_1Cu_{0.2}W_{0.1}K_1$ | 9.7 | 49.3 | 16.8 |
| 37 | $PMo_{12}V_{1.2}Cu_{0.1}Bi_{0.2}Tl_{0.5}K_1$ | 10.3 | 50.2 | 16.3 |
| 38 | $P_{1.3}Mo_{12}V_1Cu_{0.2}Se_{0.1}K_{1.5}$ | 10.2 | 51.1 | 17.3 |
| 39 | $PMo_{12}V_1Cu_{0.2}Aℓ_{0.1}Tl_1$ | 11.5 | 53.2 | 14.4 |
| 40 | $PMo_{12}V_1Cu_{0.2}As_{0.1}B_{0.1}Rb_{0.5}$ | 12.8 | 50.3 | 14.2 |
| 41 | $PMo_{12}V_{0.8}Cu_{0.2}Pr_{0.5}Rb_1$ | 10.6 | 57.8 | 14.2 |
| 42 | $P_{1.5}Mo_{12}V_1Cu_{0.2}Nd_{0.5}Cs_1$ | 12.8 | 53.8 | 15.9 |
| 43 | $PMo_{12}V_1Cu_{0.2}La_{0.5}Cs_{0.5}Tl_{0.5}$ | 11.9 | 54.4 | 15.3 |
| 44 | $PMo_{12}V_1Cu_{0.2}Ca_{0.5}Rb_{0.5}Tl_{0.5}$ | 10.8 | 53.4 | 16.9 |
| 45 | $PMo_{12}V_1Cu_{0.1}Ce_{0.5}Cs_{0.5}Tl_{0.5}$ | 11.2 | 54.9 | 15.6 |
| 46 | $PMo_{12}V_1Cu_{0.2}Co_{0.1}Rb_{0.5}$ | 9.7 | 50.0 | 18.2 |

Example 47

Using the catalyst ($PMo_{12}V_1Cu_{0.1}Ce_{0.5}Cs_{0.5}Tl_{0.5}$) prepared in Example 45, catalytic reaction was continuously performed for 1,000 hours under substanthe same conditions as in Example 1. It was found that the catalyst was not deteriorated, and that the conversion of isobutane, the selectivity for methacrylic acid and the selectivity for methacrolein were 10.9 %, 56.3 % and 15.3 %, respectively.

Example 48

A catalytic reaction was performed under substantially the same conditions as in Example 2, except that the catalyst ($PMo_{12}V_1Cu_{0.2}As_{0.1}B_{0.1}Rb_{0.5}$) prepared in Example 40 and a U-tube having an inner diameter of 6 mm as a reaction were used, and that the reaction pressure and the reaction temperature were changed to 0.3 MPa and 280 °C, respectively. 100 Hours later, the resultant gaseous reaction mixture was subjected to analysis by means of gas chromatography. It was found that 6.2 % of isobutane was converted, and that the selectivity for methacrylic acid and the selectivity for methacrolein were 50.3 % and 22.4 %, respectively. A separate catalytic reaction was continuously performed for 1,000 hours under substantially the same conditions as above. It was found that the catalyst was not deteriorated, and that the conversion of isobutane, the selectivity for methacrylic acid and the selectivity for methacrolein were 6.1 %, 51.1 % and 20.9 %, respectively.

Example 49

Using the catalyst ($PMo_{12}V_{1.2}Cu_{0.1}Bi_{0.2}Tl_{0.5}K_1$) prepared in Example 37, catalytic reaction was performed under substantially the same conditions as in Example 48. 100 Hours later, the resultant gaseous reaction mixture was subjected to analysis by means of gas chromatography. It was found that 6.0 % of isobutane was converted, and that the selectivity for methacrylic acid and the selectivity for methacrolein were 48.2 % and 24.5 %, respectively. A separate catalytic reaction was continuously performed for 3,000 hours under substantially the same conditions as above. No deterioration of the catalyst was observed, and the conversion of isobutane, the selectivity for methacrylic acid and the selectivity for methacrolein were 6.0 %, 47.1 % and 23.8 %, respectively.

Example 50

To an aqueous solution of molybdovanadophosphoric acid were added cuprous chloride, thallium nitrate and rubidium nitrate, thereby obtaining a slurry. The slurry was concentrated and evaporated to dryness, followed by drying at 130 °C for 10 hours. Then, the dried product was pulverized to obtain a powder having a composition represented by the formula: $PMo_{12}V_1Cu_{0.2}Tl_{0.5}Rb_{0.5}$. Separately, silica beads of from 100 to 200 mesh (micro bead silica gel 1,000A: produced by Fuji Debison, Japan) were calcined at 700 °C for 3 hours and then impregnated with boric acid and arsenic acid. The impregnated beads were calcined at 500 °C for 2 hours to obtain a carrier of silica having 0.03 % by weight of B and 0.03 % by weight of As carried thereon. A small amount of water was applied to the carrier, thereby wetting the carrier with the water. 100 g of the wetted carrier was placed on a rotating dish of a rolling type granulator, and 100 g of the above-mentioned powder was sprinkled over the carrier. Mixing was performed for 1 hour, thereby obtaining a carrier having the powdery composition penetrated deeply in the pores thereof and thus carried thereon. The resultant composite was impregnated with pyridine and then dried at 120 °C. Subsequently, the dried composite was calcined under a nitrogen stream at 450 °C for 3 hours and further in the air at 350 °C for 1 hour to obtain a catalyst. Reaction was performed at 320 °C in the presence of this catalyst using a fluidized-bed reactor having an internal volume of 400 ml while feeding a mixed gas of 30 % by mole of isobutane, 50 % by mole of air and 20 % by mole of steam at a linear velocity of 20 cm/sec for a contact time of 3.6 seconds. 20 Hours later, the gaseous reaction mixture was subjected to analysis by gas chromatography. It was found that 9.8 % of isobutane was converted, and that the selectivity for methacrylic acid and the selectivity for methacrolein were 48.3 % and 17.5 %, respectively. At this stage, the reduction degree of the catalyst corresponded to an increase of 0.8 electron per heteropoly acid.

Example 51

The catalyst ($PMo_{12}V_1Cu_{0.1}Ce_{0.5}Cs_{0.5}Tl_{0.5}$) as prepared in Example 45 was molded into granules having a diameter of 1 mm and a length of 5 mm using an extrusion molding machine. The granules were charged into a fixed bed reactor of SUS steel having an inner diameter of 15 mm and a height of 1.8 m. Through the mantle of the reactor, a heat transfer medium heated at 320 °C was circulated. To the reactor was fed a mixed gas of 30 % by mole of isobutane, 15 % by mole of oxygen, 20 % by mole of steam and 35 % by mole of nitrogen for a contact time of 3.6 seconds. The reaction pressure was maintained at 0.4MPa. 100 Hours later, the gaseous reaction mixture was subjected to analysis by gas chromatography. It was found that 10.3 % of isobutane was converted, and that the selectivity for methacrylic acid and the selectivity for methacrolein were 55.2 % and 17.3 %, respectively. Thereafter, a mixed gas of 30 % by mole of isobutane, 0.6 % by mole of methacrolein, 15 % by mole of oxygen, 20 % by mole of steam and 34.4 % by mole of nitrogen was reacted. Methacrolein used in the above reaction was prepared by introducing the gaseous reaction mixture formed in the reaction into a quenching tower and then into a methacrolein absorber tower in this order, isolating methacrolein from the resultant condensed liquid and the resultant absorbed liquid, and purifying the isolated methacrolein. As compared to the case where no methacrolein was supplied, the conversion of isobutane was decreased and found to be 9.0 %; however, the selectivity for methacrylic acid was improved and found to be 65.2 %. About 60 % of methacrolein was converted to methacrylic acid.

Industrial Applicability

According to the process of the present invention, it is possible to produce methacrylic acid and methacrolein in one step at low cost from isobutane which is abundant and available at low cost. Moreover, the catalyst to be used in the present process maintains the catalytic activity stably for a prolonged period of time. Therefore, the process of the present invention is extremely advantageous from the viewpoint of commercial production of methacrylic acid.

**Claims**

1. A process for the preparation of methacrylic acid and methacrolein, which comprises contacting at a temperature of 240° to 350 °C in a reactor a mixed gas comprising isobutane and molecular oxygen in the vapor phase with a catalyst comprising a composition containing a heteropoly acid having P and/or As as a central element or central elements and Mo as a coordinating element, said composition being represented by the formula:

   $$A_a Mo_{12} B_b C_c D_d O_e \quad (1)$$

   wherein A represents P and/or As; Mo represents molybdenum; B represents V and/or Cu; C represents at least one member selected from the group consisting of an alkali metal, an alkaline earth metal and Tl; D represents at least one member selected from the group consisting of Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr and Nd; O represents oxygen; and a, b, c, d and e are numbers, respectively, representing relative atomic proportions of A, B, C, D and O, wherein a, b, c and d are, respectively, in the ranges of from 0.5 to 3, from 0.01 to 3, from 0.01 to 3 and from 0 to 3, and e is the number of oxygens required to satisfy the valence and relative atomic proportion requirements of the elements present, thereby obtaining a gaseous reaction mixture comprising methacrylic acid and methacrolein.

2. The process according to claim 1, wherein the index d of formula (1) is in the range of from 0.01 to 3.

3. The process according to any one of claim 1 or 2, wherein B of formula (1) is V.

4. The process according to any one of claims 1 to 3, wherein B of formula (1) is Cu.

5. The process according to any one of claims 1 to 4, wherein said alkali metal is at least one member selected from the group consisting of Li, Na, K, Rb and Cs.

6. The process according to any one of claims 1 to 4, wherein said alkaline earth metal is at least one member selected from the group consisting of Mg, Ca, Sr and Ba.

7. The process according to any one of claims 1 to 6, wherein the index b of formula (1) is in the range of from 0.05 to 2.

8. The process according to any one of claims 1 to 7, wherein the index c of formula (1) is in the range of from 0.1 to 2.

9. The process according to any one of claims 2 to 8, wherein the index d of formula (1) is in the range of from 0.05 to 1.

**10.** The process according to any one of claims 1 to 9, wherein said temperature is in the range of from 270° to 320 °C.

**11.** The process according to any one of claims 1 to 10, wherein said gas has an isobutane content of from 10 to 80 % by mole.

**12.** The process according to claim 11, wherein the isobutane content of the gas is in the range of from 20 to 60 % by mole.

**13.** The process according to any one of claims 1 to 12, wherein the molar ratio of oxygen to isobutane in the gas is in the range of from 0.05:1 to 1:1.

**14.** The process according to claim 13, wherein the molar ratio of oxygen to isobutane in the gas is in the range of from 0.1:1 to 0.6:1.

**15.** The process according to any one of claims 1 to 14, wherein said gas contains steam.

**16.** The process according to claim 15, wherein the molar ratio of steam to isobutane in the gas is in the range of from 1:5 to 5:1.

**17.** The process according to claim 16, wherein the molar ratio of steam to isobutane in the gas is in the range of from 1:3 to 3:1.

**18.** The process according to any one of claims 1 to 17, wherein the contact time for the contacting of said gas with said catalyst is from 0.1 to 10 seconds.

**19.** The process according to claim 18, wherein the contact time for the contacting of said gas with said catalyst is from 0.5 to 5 seconds.

**20.** The process according to any one of claims 1 to 19, wherein said gas contains an inert gas.

**21.** The process according to any one of claims 1 to 20, wherein the methacrylic acid is separated from said gaseous reaction mixture, and the methacrolein is recycled to the reactor together with the unreacted isobutane.

**Patentansprüche**

**1.** Verfahren zur Herstellung von Methacrylsäure und Methacrolein, welches das in Kontakt bringen eines Isobutan und molekularen Sauerstoff enthaltenden Mischgases in der Dampfphase bei einer Temperatur von 240 bis 350 °C in einem Reaktor mit einem Katalysator umfaßt, welcher eine Heteropolysäure mit P und/oder As als Zentralelement oder Zentralelemente und Mo als Koordinationselement enthaltende Zusammensetzung umfaßt, wodurch eine Methacrylsäure und Methacrolein enthaltende gasförmige Reaktionsmischung erhalten wird, wobei die Zusammensetzung durch die Formel dargestellt ist:

$$A_a \, Mo_{12} \, B_b \, C_c \, D_d \, O_e \qquad (1),$$

worin A P und/oder As darstellt, Mo Molybdän darstellt, B V und/oder Cu darstellt, C mindestens ein Mitglied ausgewählt aus der Gruppe, bestehend aus Alkalimetallen, Erdalkalimetallen und Tl, darstellt, D mindestens ein Mitglied ausgewählt aus der Gruppe, bestehend aus Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr und Nd, darstellt, O Sauerstoff darstellt, und a, b, c, d und e jeweils Zahlen sind, welche die relativen atomaren Anteile von A, B, C, D und O darstellen, wobei a, b, c und d jeweils im Bereich von 0,5 bis 3, von 0,01 bis 3, von 0,01 bis 3 bzw. von 0 bis 3 liegen, und e die Anzahl der Sauerstoffatome ist, welche nötig ist, um den Erfordernissen bezüglich der Wertigkeit und des relativen atomaren Anteils der vorhandenen Elemente zu genügen.

**2.** Verfahren gemäß Anspruch 1, worin der Index d der Formel (1) im Bereich von 0,01 bis 3 liegt.

14

EP 0 425 666 B1

**3.** Verfahren gemäß Anspruch 1 oder 2, worin B der Formel (1) V ist.

**4.** Verfahren gemäß einem der Ansprüche 1 bis 3, worin B der Formel (1) Cu ist.

**5.** Verfahren gemäß einem der Ansprüche 1 bis 4, worin das Alkalimetall mindestens eines ausgewählt aus der Gruppe, bestehend aus Li, Na, K, Rb und Cs ist.

**6.** Verfahren gemäß einem der Ansprüche 1 bis 4, worin das Erdalkalimetall mindestens eines ausgewählt der Gruppe, bestehend aus Mg, Ca, Sr und Ba ist.

**7.** Verfahren gemäß einem der Ansprüche 1 bis 6, worin der Index b der Formel (1) im Bereich von 0,05 bis 2 liegt.

**8.** Verfahren gemäß einem der Ansprüche 1 bis 7, worin der Index c der Formel (1) im Bereich von 0,1 bis 2 liegt.

**9.** Verfahren gemäß einem der Ansprüche 2 bis 8, worin der Index d der Formel (1) im Bereich von 0,05 bis 1 liegt.

**10.** Verfahren gemäß einem der Ansprüche 1 bis 9, worin die Temperatur im Bereich von 270 bis 320 °C liegt.

**11.** Verfahren gemäß einem der Ansprüche 1 bis 10, worin das Gas einen Gehalt an Isobutan von 10 bis 80 mol-% besitzt.

**12.** Verfahren gemäß Anspruch 11, worin der Gehalt an Isobutan des Gases im Bereich von 20 bis 60 mol-% liegt.

**13.** Verfahren gemäß einem der Ansprüche 1 bis 12, worin das molare Verhältnis von Sauerstoff zu Isobutan im Gas im Bereich von 0,05:1 bis 1:1 liegt.

**14.** Verfahren gemäß Anspruch 13, worin das molare Verhältnis von Sauerstoff zu Isobutan im Gas im Bereich von 0,1:1 bis 0,6:1 liegt.

**15.** Verfahren gemäß einem der Ansprüche 1 bis 14, worin das Gas Dampf enthält.

**16.** Verfahren gemäß Anspruch 15, worin das molare Verhältnis von Dampf zu Isobutan im Gas im Bereich von 1:5 bis 5:1 liegt.

**17.** Verfahren gemäß Anspruch 16, worin das molare Verhältnis von Dampf zu Isobutan im Gas im Bereich von 1:3 bis 3:1 liegt.

**18.** Verfahren gemäß einem der Ansprüche 1 bis 17, worin die Kontaktzeit für das in Kontakt bringen des Gases mit dem Katalysator 0,1 bis 10 Sekunden beträgt.

**19.** Verfahren gemäß Anspruch 18, worin die Kontaktzeit für das in Kontakt bringen des Gases mit dem Katalysator 0,5 bis 5 Sekunden beträgt.

**20.** Verfahren gemäß einem der Ansprüche 1 bis 19, worin das Gas ein inertes Gas enthält.

**21.** Verfahren gemäß einem der Ansprüche 1 bis 20, worin die Methacrylsäure aus der gasförmigen Reaktionsmischung abgetrennt wird, und das Methacrolein zusammen mit nicht umgesetztem Isobutan in den Reaktor zurückgeführt wird.

**Revendications**

**1.** Procédé de préparation d'acide méthacrylique et de méthacroléïne, qui consiste à mettre en contact à une température de 240 à 350 °C dans un réacteur un gaz mixte comprenant de l'isobutane et de

15

l'oxygène moléculaire en phase vapeur avec un catalyseur comprenant une composition contenant un hétéropolyacide ayant P et/ou As comme élément central ou comme éléments centraux et Mo comme élément de coordination, la composition étant représentée par la formule:

$$A_a\ Mo_{12}\ B_b\ C_c\ D_d\ O_e \qquad (1)$$

dans laquelle A représente P et/ou As; Mo représente le molybdène, B représente V et/ou Cu; C représente au moins un élément choisi parmi un métal alcalin, un métal alcalino terreux et Tl; D représente au moins un élément choisi parmi Ag, Zn, Cd, Ti, Zr, Nb, Ta, Cr, W, Mn, Fe, Co, Ni, B, Al, Ge, Rh, Sn, Sb, Bi, Se, Te, Y, La, Ce, Pr et Nd; O représente l'oxygène; et a, b, c, d et e sont des nombres représentant respectivement les proportions atomiques relatives de A, B, C, D et O, a, b, c et d étant, respectivement, compris entre 0,5 et 3, entre 0,01 et 3, entre 0,01 et 3 et entre 0 et 3, et e est le nombre d'oxygènes exigé pour satisfaire la valence et les exigences de proportions atomiques relatives des éléments présents, de manière à obtenir un mélange réactionnel gazeux comprenant de l'acide méthacrylique et de la méthacroléïne.

2. Procédé suivant la revendication 1, dans lequel l'indice d à la formule (1) est compris entre 0,01 et 3.

3. Procédé selon l'une quelconque des revendications 1 ou 2, dans lequel B à la formule (1) est Y.

4. Procédé selon l'une quelconque des revendications 1 à 3, dans lequel B à la formule (1) est Cu.

5. Procédé selon l'une quelconque des revendications 1 à 4, dans lequel le métal alcalin est au moins un élément choisi parmi Li, Na, K, Rb et Cs.

6. Procédé selon l'une quelconque des revendications 1 à 4, dans lequel le métal alcalino terreux est au moins un élément choisi parmi Mg, Ca, Sr et Ba.

7. Procédé selon l'une quelconque des revendications 1 à 6, dans lequel l'indice b à la formule (1) est compris entre 0,05 et 2.

8. Procédé selon l'une quelconque des revendications 1 à 7, dans lequel l'indice c à la formule (1) est compris entre 0,1 et 2.

9. Procédé selon l'une quelconque des revendications 2 à 8, dans lequel l'indice d à la formule (1) est compris entre 0,05 et 1.

10. Procédé selon l'une quelconque des revendications 1 à 9, dans lequel la température est comprise entre 270 et 320°C.

11. Procédé selon l'une quelconque des revendications 1 à 10, dans lequel le gaz a une teneur en isobutane comprise entre 10 et 80 % en mole.

12. Procédé selon la revendication 11, caractérisé en ce que la teneur en isobutane du gaz est comprise entre 20 et 60 % en mole.

13. Procédé selon l'une quelconque des revendications 1 à 12, dans lequel le rapport molaire de l'oxygène à l'isobutane du gaz est compris entre 0,05:1 et 1:1.

14. Procédé suivant la revendication 13, dans lequel le rapport molaire de l'oxygène à l'isobutane du gaz est compris entre 0,1:1 et 0,6:1.

15. Procédé selon l'une quelconque des revendications 1 à 14, dans lequel le gaz contient de la vapeur d'eau.

16. Procédé suivant la revendication 15, dans lequel le rapport molaire de la vapeur à l'isobutane du gaz est compris entre 1:5 et 5:1.

**17.** Procédé suivant la revendication 16, dans lequel le rapport molaire de la vapeur d'eau à l'isobutane du gaz est compris entre 1:3 et 3:1.

**18.** Procédé selon l'une quelconque des revendications 1 à 17, dans lequel la durée de contact pour la mise en contact du gaz avec le catalyseur est comprise entre 0,1 à 10 secondes.

**19.** Procédé suivant la revendication 18, dans lequel la durée de contact pour la mise en contact du gaz avec le catalyseur est comprise entre 0,5 et 5 secondes.

**20.** Procédé selon l'une quelconque des revendications 1 à 19, dans lequel le gaz contient un gaz inerte.

**21.** Procédé selon l'une quelconque des revendications 1 à 20, dans lequel l'acide méthacrylique est séparé du mélange gazeux de réaction et la méthacroléïne est recyclée au réacteur en même temps que l'isobutane inaltéré.